# EUROPÄISCHE PATENTSCHRIFT

(11) **EP 1 339 764 B1**
(45) Veröffentlichungstag und Bekanntmachung des Hinweises auf die Patenterteilung: **01.04.2015**
(21) Anmeldenummer: 01985828.1
(22) Anmeldetag: 28.11.2001
(51) Int. Cl.: C08F 220/58, C08F 291/00, C08F 290/00, C08F 265/00, C08L 51/00, C08F 265/10

(54) **SILIZIUMMODIFIZIERTE KAMMPOLYMERE AUF BASIS VON ACRYLOYLDIMETHYLTAURINSÄURE (2-ACRYLAMIDO-2-METHYL-1-PROPANSULFONSÄURE)**
SILICON-MODIFIED COMB POLYMERS BASED ON ACRYLOYLDIMETHYL TAURINE ACID
POLYMERES EN PEIGNE MODIFIES PAR SILICIUM, A BASE D'ACIDE D'ACRYLOYLDIMETHYLTAURINE

(30) Priorität: 01.12.2000 DE 10059831
(43) Veröffentlichungstag der Anmeldung: 03.09.2003
(73) Patentinhaber: Clariant Produkte (Deutschland) GmbH, 65929 Frankfurt am Main (DE)
(72) Erfinder: MORSCHHÄUSER, Roman, 55122 Mainz (DE); GLAUDER, Jan, 65812 Bad Soden (DE); LÖFFLER, Matthias, 65527 Niedernhausen (DE); RUDLOFF, Susan, 06127 Merseburg (DE); KLEIN, Sonja, 65795 Hattersheim (DE)
(86) Internationale Anmeldenummer: PCT/EP2001/013858
(87) Internationale Veröffentlichungsnummer: WO 2002/044225

(56) Entgegenhaltungen:
- EP-A- 0 356 241
- EP-A- 0 503 853
- EP-A- 0 815 844
- EP-A- 0 815 845
- EP-A- 1 083 184
- WO-A-00/12588
- WO-A-99/04750
- DE-A- 19 907 587
- DE-A- 19 907 715
- DE-A- 19 951 877
- FR-A- 2 791 558
- US-A- 4 606 933
- US-A- 5 368 850

## Beschreibung

Die vorliegende Erfindung betrifft siliziummodifizierte auf Basis von Acryloyldimethyltaurinsäure bzw. Acryloyldimethyltauraten.

In den letzten Jahren erlangten wasserlösliche Polymere eine immer größer werdende Bedeutung in Industrie und Wissenschaft. Polyelektrolyte nehmen dabei mengenmäßig einen sehr großen Teil der jährlichen Gesamtproduktion ein. Sie finden z.B. Anwendung in der Papierverarbeitung, der Waschmittelindustrie, der Textilverarbeitung, der Erdölgewinnung oder als wichtige Kosmetikrohstoffe.

Im kosmetischen Bereich kommt Polyelektrolyten eine tragende Rolle zu. Neben wasserlöslichen, oberflächenaktiven Stoffen gibt es in diesem Bereich einen hohen Bedarf an wasser- und ölverdickenden Systemen. Derartige Verdicker, insbesondere die auf Basis der Polyacrylsäure hergestellten "Superabsorber", sind seit ihrer Entwicklung in den 70iger Jahren aus dem Hygienebereich nicht mehr wegzudenken. In ihren vernetzten Varianten werden teil- oder vollneutralisierte Polyacrylsäuren und deren wasserlösliche Copolymere in vielen Kosmetikformulierungen als Konsistenzgeber eingesetzt. Die Vielfalt der möglichen Strukturen und die damit verbundenen vielfältigen Anwendungsmöglichkeiten drücken sich nicht zuletzt in einer Vielzahl von Patenten aus, die seit Mitte der 70iger Jahre weltweit angemeldet wurden.

In den 90iger Jahren wurden neuartige Verdicker auf Basis von 2-Acrylamido-2-methyl-1-propansulfonsäure (AMPS) bzw. deren Salzen in den Markt eingeführt (EP 816 403 DE19907587, WO9904750 und WO 98/ 00094). Sowohl als Homo- als auch in Form der Copolymere (^{®}Aristoflex AVC, Clariant GmbH) sind derartige Verdicker den entsprechenden Polycarboxylaten (Carbopole) in vieler Hinsicht überlegen. Beispielsweise zeigen Verdickersysteme auf Basis von AMPS hervorragende Eigenschaften in pH-Bereichen unterhalb von pH 6, also in einem pH-Bereich, in dem mit herkömmlichen Polycarboxylat-Verdickern nicht mehr gearbeitet werden kann. Zudem führt die Mikrogelstruktur solcher Verdicker zu einem besonders angenehmen Hautgefühl. Die leichte Verarbeitbarkeit und das günstige toxikologische Profil des Hauptmonomeren verleihen diesen Verdickern ein hohes Anwendungspotential.

Im Laufe der letzten Jahre drängten Vertreter eines neuen Verdickerkonzeptes auf den Markt. Hierbei wurden in einem Polymer zwei unterschiedliche Eigenschaften kombiniert und damit neue Anwendungsfelder erschlossen. Verdickende Emulgatoren oder Dispergatoren sind nur zwei Beispiele dieser neuen Substanzklasse. Als Markennamen können die Pemulene^{®} TR-1 und TR-2 von BF-Goodrich oder die Aculyn^{®}-Typen von Rohm und Haas genannt werden. Alle bisherigen Varianten basieren auf hydrophob modifizierten Varianten der herkömmlichen Polyacrylate.

Ziel dieser Erfindung war die Synthese einer neuen Polymerklasse basierend auf dem Konzept der molekularen Eigenschaftskombination, die dem Formulierer die Möglichkeit geben soll, Silikonöl-haltige wässrige Systeme zu stabilisieren bzw. zu verdicken.

Durch radikalische Copolymerisation von Acryloyldimethyltaurinsäure (AMPS) bzw. Acryloyldimethyltauraten, gegebenenfalls in Gegenwart weiterer Comonomerer oder polymerer Additive, und geeigneten vinylischen mono- oder polyfunktionellen Silikonderivaten, gelang sowohl die Synthese vernetzter als auch unvemetzter Strukturen mit interessanten anwendungstechnischen Eigenschaften. Insbesondere ist mit den neu entwickelten Strukturen die Stabilisierung von silikonhaltigen Emulsionen mit sehr hohen Anteilen an Silikonöl (> 30 %) möglich. Vorteilhafterweise zeigen derartige Emulsionen ein sehr angenehmes Hautgefühl.

Gegenstand der Erfindung sind wasserlösliche oder wasserquellbare Copolymere, erhältlich durch radikalische Copolymerisation von
A) Acryloyldimethyltaurinsäure und/oder Acryloyldimethyltauraten,
B) gegebenenfalls einem oder mehreren olefinisch ungesättigten, gegebenenfalls vernetzenden, Comonomeren, die wenigstens ein Sauerstoff-, Stickstoff-, Schwefel- oder Phosphoratom aufweisen und ein Molekulargewicht kleiner 500 g/mol besitzen, und ausgewählt sind aus ungesättigten Carbonsäuren und deren Anhydriden und Salzen, sowie deren Estern mit aliphatischen, olefinischen, cycloaliphatischen, arylaliphatischen oder aromatischen Alkoholen mit einer Kohlenstoffzahl von 1 bis 22, offenkettigen N-Vinylamiden, bevorzugt N-Vinylformamid (VIFA), N-Vinylmethylformamid, N-Vinylmethylacetamid (VIMA) und N-Vinylacetamid; cyclischen N-Vinylamiden (N-Vinyllactame) mit einer Ringgröße von 3 bis 9, bevorzugt N-Vinylpyrrolidon (NVP) und N-Vinylcaprolactam; Amiden der Acryl- und Methacrylsäure, bevorzugt Acrylamid, Methacrylamid, N,N-Dimethyl-acrylamid, N,N-Diethylacrylamid und N,N-Diisopropylacrylamid; alkoxylierten Acryl- und Methacrylamiden, bevorzugt Hydroxyethylmethacrylat, Hydroxymethylmethacrylamid, Hydroxyethylmethacrylamid, Hydroxypropylmethacrylamid und Bernsteinsäuremono-[2-(methacryloyloxy)-ethylester]; N,N-Dimethylaminomethacrylat und Diethylaminomethylmethacrylat; Acryl-und Methacrylamidoglykolsäure; 2- und 4-Vinylpyridin; Vinylacetat; Methacrylsäureglycidylester; Styrol; Acrylnitril; Vinylchlorid; Stearylacrylat; Laurylmethacrylat; Vinylidenchlorid; und/oder Tetrafluorethylen; anorganischen Säuren und deren Salzen und Estern, bevorzugt Vinylphosphonsäure, Vinylsulfonsäure, Allylphosphonsäure und Methallylsulfonsäure,
C) einer oder mehreren mindestens monofunktionellen, zur radikalischen Polymerisation befähigten, siliziumhaltigen Komponente(n), wobei mindestens eine siliziumhaltige Komponente eine Verbindung ausgewählt aus den Formeln 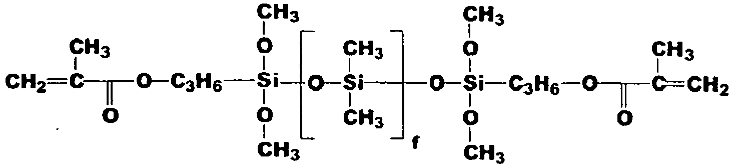 mit f=2 bis 500 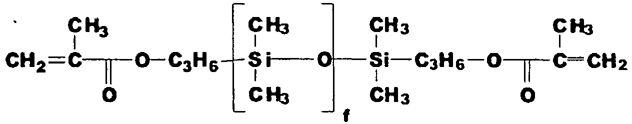 mit f=2 bis 500
   und/oder 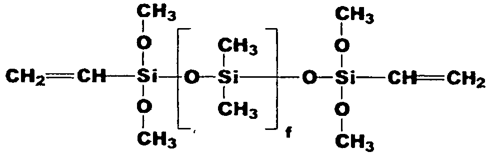
mit f=2 bis 500 ist, wobei die Copolymerisation
in Gegenwart mindestens eines polymeren Additivs mit zahlenmittleren Molekulargewichten von 200 g/mol bis 10⁹ g/mol ausgewählt aus Homo- und Copolymeren aus N-Vinylformamid, N-Vinylacetamid, N-Vinylpyrrolidon, Acryloyldimethyltaurinsäure, N-Vinylcaprolactam, N-Vinylmethylacetamid, Acrylamid, Acrylsäure, Methacrylsäure, N-Vinylmorpholid, Hydroxyethylmethacrylat, Diallyldimethylammoniumchlorid (DADMAC) und/oder [2-(Methacryloyloxy)ethyl]trimethylammoniumchlorid (MAPTAC); erfolgt.

Die erfindungsgemäßen Copolymere besitzen bevorzugt ein Molekulargewicht von 10³ g/mol bis 10⁹ g/mol, besonders bevorzugt von 10⁴ bis 10⁷ g/mol, insbesondere bevorzugt 5*10⁴ bis 5*10⁶ g/mol.

Bei den Acryloyldimethyltauraten kann es sich um die anorganischen oder organischen Salze der Acryloyldimethyltaurinsäure handeln. Bevorzugt werden die Li⁺-, Na⁺-, K⁺-, Mg⁺⁺-, Ca⁺⁺-, Al⁺⁺⁺- und/oder NH₄⁺-Salze. Ebenfalls bevorzugt sind die Monoalkylammonium-, Dialkylammonium-, Trialkylammonium- und/oder Tetraalkylammoniumsalze, wobei es sich bei den Alkylsubstituenten der Amine unabhängig voneinander um (C₁-C₂₂)-Alkylreste handeln kann, die gegebenenfalls mit bis zu 3 (C₂-C₁₀)-Hydroxyalkylgruppen besetzt sein können. Weiterhin sind auch ein bis dreifach ethoxylierte Ammoniumverbindungen mit unterschiedlichem Ethoxylierungsgrad bevorzugt. Es sollte angemerkt werden, dass auch Mischungen von zwei- oder mehreren der oben genannten Vertreter im Sinne der Erfindung sind.

Der Neutralisationsgrad der Acryloyldimethyltaurinsäure kann zwischen 0 und 100 % betragen, besonders bevorzugt ist ein Neutralisationsgrad von oberhalb 80 %.

Bezogen auf die Gesamtmasse der Copolymere beträgt der Gehalt an Acryloyldimethyltaurinsäure bzw. Acryloyldimethyltauraten mindestens 0,1 Gew.-%, bevorzugt 20 bis 99,5 Gew.-%, besonders bevorzugt 50 bis 98 Gew.-%.

Als Comonomere B) können alle olefinisch ungesättigten Monomere eingesetzt werden, deren Reaktionsparameter eine Copolymerisation mit Acryloyldimethyltaurinsäure und/oder Acryloyldimethyltauraten in den jeweiligen Reaktionsmedien erlauben.

Als Comonomere B) bevorzugt sind ungesättigte Carbonsäuren und deren Anhydride und Salze, sowie deren Ester mit aliphatischen, olefinischen, cycloaliphatischen, arylaliphatischen oder aromatischen Alkoholen mit einer Kohlenstoffzahl von 1 bis 22. Als ungesättigte Carbonsäuren besonders bevorzugt sind Acrylsäure, Methacrylsäure, Styrolsulfonsäure, Maleinsäure, Fumarsäure, Crotonsäure, Itaconsäure und Seneciosäure. Als Gegenionen bevorzugt sind Li⁺, Na⁺, K⁺, Mg⁺⁺, Ca⁺⁺, Al⁺⁺⁺, NH₄⁺, Monoalkylammonium-, Dialkylammonium-, Trialkylammonium- und/oder Tetraalkylammoniumreste, wobei es sich bei den Alkylsubstituenten der Amine unabhängig voneinander um (C₁-C₂₂)-Alkylreste oder (C₂-C₁₀)-Hydroxyalkylreste handelt. Zusätzlich können auch ein bis dreifach ethoxylierte Ammonium-verbindungen mit unterschiedlichem Ethoxylierungsgrad Anwendung finden.

Der Neutralisationsgrad der Carbonsäuren kann zwischen 0 und 100 % betragen, besonders bevorzugt ist ein Neutralisationsgrad von oberhalb 80 %.

Als Comonomere weiterhin bevorzugt sind offenkettige N-Vinylamide, bevorzugt N-Vinylformamid (VIFA), N-Vinylmethylformamid, N-Vinylmethylacetamid (VIMA) und N-Vinylacetamid; cyclische N-Vinylamide (N-Vinyllactame) mit einer Ringgröße von 3 bis 9, bevorzugt N-Vinylpyrrolidon (NVP) und N-Vinylcaprolactam; Amide der Acryl- und Methacrylsäure, bevorzugt Acrylamid, Methacrylamid, N,N-Dimethylacrylamid, N,N-Diethylacrylamid und N,N-Diisopropylacrylamid; alkoxylierte Acryl-und Methacrylamide, bevorzugt Hydroxyethylmethacrylat, Hydroxymethylmethacrylamid, Hydroxyethylmethacrylamid, Hydroxypropylmethacrylamid und Bernsteinsäuremono-[2-(methacryloyloxy)-ethylester]; N,N-Dimethylaminomethacrylat und Diethylaminomethylmethacrylat; Acryl- und Methacrylamidoglykolsäure; 2- und 4-Vinylpyridin; Vinylacetat; Methacrylsäureglycidylester; Styrol; Acrylnitril; Vinylchlorid; Stearylacrylat; Laurylmethacrylat; Vinylidenchlorid; und/oder Tetrafluorethylen. Als Comonomere B) ebenfalls geeignet sind anorganische Säuren und deren Salze und Ester. Bevorzugte Säuren sind Vinylphosphonsäure, Vinylsulfonsäure, Allylphosphonsäure und Methallylsulfonsäure.

Der Gewichtsanteil der Comonomere B), bezogen auf die Gesamtmasse der Copolymere, kann 0 bis 99,8 Gew.-% betragen und beträgt bevorzugt 0,5 bis 80 Gew.-%, besonders bevorzugt 2 bis 50 Gew.-%. Als polymerisationsfähige, silikonhaltige Komponente C) sind alle mindestens einfach olefinisch ungesättigten Verbindungen geeignet, die unter den jeweils gewählten Reaktionsbedingungen mit Acryloyldimethyltaurinsäure bzw. Acryloyldimethyltaurat und gegebenenfalls weiteren Comonomeren zur radikalischen Copolymerisation befähigt sind. Dabei muss die Verteilung der einzelnen silikonhaltigen Monomere über die entstehenden Polymerketten hinweg nicht notwendigerweise statistisch erfolgen. Auch die Ausbildung von beispielsweise block- (auch multiblock-) oder gradientenartigen Strukturen ist im Sinne der Erfindung. Kombinationen von zwei oder mehreren unterschiedlichen silikonhaltigen Vertretern sind auch möglich. Die Verwendung von silikonhaltigen Komponenten mit zwei oder mehr polymerisationsaktiven Gruppen führt zum Aufbau verzweigter oder vernetzter Strukturen.

Bevorzugte siliziumhaltige Komponenten sind solche gemäß Formel (I)

R¹-Z-[(Si(R³R⁴)-O-)_{w}-(Si(R⁵R⁶)-O)ₓ-]-R² (I)

Dabei stellt R¹ eine polymerisationsfähige Funktion aus der Gruppe der vinylisch ungesättigten Verbindungen dar, die zum Aufbau polymerer Strukturen auf radikalischem Wege geeignet ist. Bevorzugt stellt R¹ einen Vinyl-, Allyl-, Methallyl-, Methylvinyl-, Acryl- (CH₂=CH-CO-), Methacryl- (CH₂=C[CH_{3]}-CO-), Crotonyl-, Senecionyl-, Itaconyl-, Maleinyl-, Fumaryl- oder Styrylrest dar.

Zur Anbindung der silikonhaltigen Polymerkette an die reaktive Endgruppe R¹ ist teilweise eine geeignete chemische Brücke Z erforderlich. Bevorzugte Brücken Z sind -O-, -((C₁ - C₅₀)Alkylen)-, -((C₆ - C₃₀) Arylen)-, -((C₅ - C₈) Cycloalkylen)-, -((C₁-C₅₀)Alkenylen)-, -(Polypropylenoxid)ₙ-, -(Polyethylenoxid)ₒ-, -(Polypropylenoxid)ₙ(Polyethylenoxid)ₒ-, wobei n und o unabhängig voneinander Zahlen von 0 bis 200 bedeuten und die Verteilung der EO/PO-Einheiten statistisch oder blockförmig sein kann. Weiterhin geeignet als Brückengruppierungen Z sind -((C₁ - C₁₀)Alkyl)-(Si(OCH₃)₂)- und -(Si(OCH₃)₂)-.

Der polymere Mittelteil wird durch silikonhaltige Wiederholungseinheiten repräsentiert.

Die Reste R³, R⁴, R⁵ und R⁶ bedeuten unabhängig voneinander -CH₃, -O-CH₃, -C₆H₅ oder -O-C₆H₅.

Die Indizes w und x repräsentieren stöchiometrische Koeffizienten, die unabhängig voneinander 0 bis 500, bevorzugt 10 bis 250, betragen.

Die Verteilung der Wiederholungseinheiten über die Kette hinweg kann nicht nur rein statistisch, sondern auch blockartig, alternierend oder gradientenartig sein kann.

R² kann einerseits einen aliphatischen, olefinischen, cycloaliphatischen, arylaliphatischen oder aromatischen (C₁ - C₅₀)-Kohlenwasserstoffrest symbolisieren (linear oder verzweigt) oder -OH, -NH₂, -N(CH₃)₂, -R⁷ oder für die Struktureinheit

[-Z-R¹] stehen. Die Bedeutung der beiden Variablen Z und R¹ wurde bereits erklärt. R⁷ steht für weitere Si-haltige Gruppierungen. Bevorzugte R⁷-Reste sind -O-Si(CH₃)₃, -O-Si(Ph)₃, -O-Si(O-Si(CH₃)₃)₂CH₃) und -O-Si(O-Si(Ph)₃)₂Ph).

Wenn R² ein Element der Gruppe [-Z-R¹] darstellt, handelt es sich um difunktionelle, Monomere, die zur Vernetzung der entstehenden Polymerstrukturen herangezogen werden können.

Formel (I) beschreibt nicht nur vinylisch funktionalisierte, silikonhaltige Polymerspezies mit einer polymertypischen Verteilung, sondern auch definierte Verbindungen mit diskreten Molekulargewichten.

Als siliziumhaltige Komponenten (c) sind in mindestens eine siliziumhaltige Komponente eine Verbindung ausgewählt aus den Formeln: 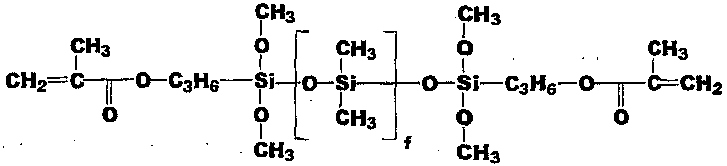 Methacryloxyproplydimethylsilyl endgeblockte Polydimethylsiloxane (f = 2 bis 500) 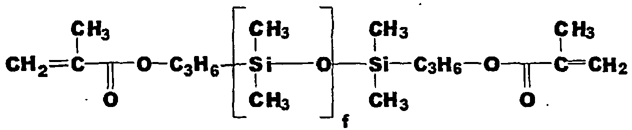 Methacryloxypropyl endgeblockte Polydimethylsiloxane (f = 2 bis 500 bis) 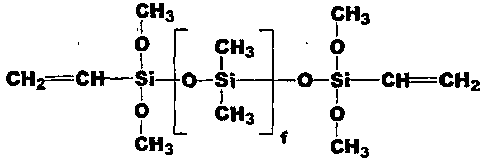 Vinyldimethoxysilyl endgeblockte Polydimethylsiloxane (f = 2-500)

Der Gewichtsanteil der Comonomeren D) beträgt, bezogen auf die Gesamtmasse der Copolymere, 0,1 bis 99,9 Gew.-%, bevorzugt 0,1 bis 50 Gew.-%, besonders bevorzugt 0,2 bis 40 Gew.-% und insbesondere bevorzugt 0.5 bis 30 Gew.-%.

Die Copolymerisation wird in Gegenwart mindestens eines polymeren Additivs D) durchgeführt wird, wobei das Additiv D) vor der eigentlichen Copolymerisation dem Polymerisationsmedium ganz- oder teilweise gelöst zugegeben wird. Die Verwendung von mehreren Additiven D) ist ebenfalls erfindungsgemäß. Vernetzte Additive D) können ebenfalls verwendet werden.

Die Additive D) bzw. deren Mischungen müssen lediglich ganz oder teilweise im gewählten Polymerisationsmedium löslich sein.

Während des eigentlichen Polymerisationsschrittes hat das Additiv D) mehrere Funktionen. Einerseits verhindert es im eigentlichen Polymerisationsschritt die Bildung übervernetzter Polymeranteile im sich bildenden Copolymerisat und andererseits wird das Additiv D) gemäß dem allgemein bekannten Mechanismus der Pfropf-Copolymerisation statistisch von aktiven Radikalen angegriffen. Dies führt dazu, dass je nach Additiv D) mehr oder weniger große Anteile davon in die Copolymere eingebaut werden. Zudem besitzen geeignete Additive D) die Eigenschaft, die Lösungsparameter der sich bildenden Copolymere während der radikalischen Polymerisationsreaktion derart zu verändern, dass die mittleren Molekulargewichte zu höheren Werten verschoben werden. Verglichen mit analogen Copolymeren, die ohne den Zusatz der Additive D) hergestellt wurden, zeigen solche, die unter Zusatz von Additiven D) hergestellt wurden, vorteilhafterweise eine signifikant höhere Viskosität in wässriger Lösung.

Bevorzugt als Additive D) sind in Wasser und/oder Alkoholen lösliche Homo- und Copolymere. Unter Copolymeren sind dabei auch solche mit mehr als zwei verschiedenen Monomertypen zu verstehen.

Besonders bevorzugt als Additive D) sind Homo- und Copolymere aus N-Vinylformamid, N-Vinylacetamid, N-Vinylpyrrolidon, Acryloyldimethyltaurinsäure, N-Vinylcaprolactam, N-Vinylmethylacetamid, Acrylamid, Acrylsäure, Methacrylsäure, N-Vinylmorpholid, Hydroxyethylmethacrylat, Diallyldimethylammoniumchlorid (DADMAC) und/oder [2-(Methacryloyloxy)ethyl]trimethylammoniumchlorid (MAPTAC) .

Insbesondere bevorzugt als Additive D) sind Polyvinylpyrrolidone (z.B. K15^{®}, K20^{®} und K30^{®} von BASF), Poly(N-Vinylformamide), Poly(N-Vinylcaprolactame) und Copolymere aus N-Vinylpyrrolidon, N-Vinylformamid und/oder Acrylsäure, die auch teilweise oder vollständig verseift sein können.

Das Molekulargewicht der Additive D) beträgt bevorzugt 10³ bis 10⁷ g/mol, besonders bevorzugt 0,5*10⁴ bis 10⁶ g/mol.

Die Einsatzmenge des polymeren Additivs D) beträgt, bezogen auf die Gesamtmasse der bei der Copolymerisation zu polymerisierenden Monomere, bevorzugt 0,1 bis 90 Gew.-%, besonders bevorzugt 1 bis 20 Gew.-% und insbesondere bevorzugt 1,5 bis 10 Gew.-%.

In einer weiteren bevorzugten Ausführungsform sind die erfindungsgemäßen Copolymere vernetzt, d.h. sie enthalten Comonomere mit mindestens zwei polymerisationsfähigen Vinylgruppen.

Bevorzugte Vernetzer sind Methylenbisacrylamid; Methylenbismethacrylamid; Ester ungesättigter Mono- und Polycarbonsäuren mit Polyolen, bevorzugt Diacrylate und Triacrylate bzw. -methacrylate, besonders bevorzugt Butandiol- und Ethylenglykoldiacrylat bzw. -methacrylat, Trimethylolpropantriacrylat (TMPTA) und Trimethylolpropantrimethacrylat (TMPTMA); Allylverbindungen, bevorzugt Allyl(meth)acrylat, Triallylcyanurat, Maleinsäurediallylester, Polyallylester, Tetraallyloxyethan, Triallylamin, Tetraallylethylendiamin; Allylester der Phosphorsäure; und/oder Vinylphosphonsäurederivate.

Besonders bevorzugt als Vernetzer ist Trimethylolpropantrimethacrylat (TMPTMA).

Der Gewichtsanteil an vernetzenden Comonomeren, bezogen auf die Gesamtmasse der Copolymere, beträgt bevorzugt bis 20 Gew.-%, besonders bevorzugt 0,05 bis 10 Gew.-% und insbesondere bevorzugt 0,1 bis 7 Gew.-%.

Als Polymerisationsmedium können alle organischen oder anorganischen Lösungsmittel dienen, die sich bezüglich radikalischer Polymerisationsreaktionen weitestgehend inert verhalten und vorteilhafterweise die Bildung mittlerer oder hoher Molekulargewichte zulassen. Bevorzugt Verwendung finden Wasser; niedere Alkohole; bevorzugt Methanol, Ethanol, Propanole, iso-, sec.- und t-Butanol, insbesondere bevorzugt t-Butanol; Kohlenwasserstoffe mit 1 bis 30 Kohlenstoffatomen und Mischungen und Emulsionen der vorgenannten Verbindungen.

Die Polymerisationsreaktion erfolgt bevorzugt im Temperaturbereich zwischen 0 und 150°C, besonders bevorzugt zwischen 10 und 100°C, sowohl bei Normaldruck als auch unter erhöhtem oder erniedrigtem Druck. Gegebenenfalls kann die Polymerisation auch unter einer Schutzgasatmosphäre, vorzugsweise unter Stickstoff, ausgeführt werden.

Zur Auslösung der Polymerisation können energiereiche elektromagnetische Strahlen, mechanische Energie oder die üblichen chemischen Polymerisationsinitiatoren, wie organische Peroxide, z.B. Benzoylperoxid, tert.-Butylhydroperoxid, Methylethylketonperoxid, Cumolhydroperoxid, Dilauroylperoxid (DLP) oder Azoinitiatoren, wie z.B. Azodiisobutyronitril (AIBN), verwendet werden.

Ebenfalls geeignet sind anorganische Peroxyverbindungen, wie z.B. (NH₄)₂S₂O₈, K₂S₂O₈ oder H₂O₂, gegebenenfalls in Kombination mit Reduktionsmitteln (z.B. Natriumhydrogensulfit, Ascorbinsäure, Eisen(II)-sulfat etc.) oder Redoxsystemen, welche als reduzierende Komponente eine aliphatische oder aromatische Sulfonsäure (z.B. Benzolsulfonsäure, Toluolsulfonsäure etc.) enthalten.

Die Polymerisationsreaktion kann z.B. als Fällungspolymerisation, Emulsionspolymerisation, Lösungspolymerisation, Substanzpolymerisation oder Gelpolymerisation geführt werden. Besonders vorteilhaft für das Eigenschaftsprofil der erfindungsgemäßen Copolymere ist die Fällungspolymerisation, bevorzugt in tert.-Butanol.

Die erfindungsgemäßen Copolymere versetzen den Anwender erstmals in die Lage, Silikonöle, gegebenenfalls in hoher Konzentration, in Wasser zu verdicken, oder aber, im Falle hochmodifizierter Varianten, Silikonöle selbst zu verdicken. Damit werden Formulierungen mit starkem Silikonölcharakter z. B. in Stick- oder Gelform möglich, die nicht die nachteilhafte flüssige Konsistenz von Ölen aufzuweisen.

Zusätzlich kann man mit den erfindungsgemäßen Polymeren Emulsionen mit herkömmlichen Ölen herstellen, die Si-haltige Emulgatoren (gegebenenfalls auch Silikonöle) enthalten, da dies die Eigenschaften von Silikonölen und herkömmlichen Ölen verbindet und zudem die Stabilität der Emulsion durch Verdickung der Phasen ermöglicht. Alle diese Emulsionen zeigen einen ausgesprochen guten Glanz, was für eine kosmetische Emulsion von großem Gewicht ist. Hervorzuheben ist hierbei das hervorragende Hautgefühl dieser Formulierungen gegenüber herkömmlichen Formulierungen. Bei Anwendungen im Bereich der Haarkosmetik fällt der angenehme Konditioniereffekt der Polymere auf, sowie die gute Kämmbarkeit und Glanz.

Die erfindungsgemäßen Copolymere können unter anderem eingesetzt werden in W/O Emulsionen, O/W Emulsionen, Hautschutzformulierungen, Shampoos, Spülungen, Kuren, dekorative Kosmetik wie Lippenstifte, Lippenpflegestifte oder Lotionen etc., Make-up wie flüssiges Make-up, cover and resist Make-up, Make-up Puder etc., Deosticks, Antiperspirantien, Duschbäder, flüssige Seife, Stückseife, Reinigungs-milch, Sonnenschutzformulierungen, Dauerwellen, Haarfarben, Haargelen, Haarsprays, um nur einige zu nennen. Im Falle von z.B. Gesichts-Make-up ist hervorzuheben, dass durch Einsatz der erfindungsgemäßen Polymere überschüssiges Hautfett aufgenommen werden kann.

Die nachfolgenden Beispiele dienen der näheren Erläuterung der Erfindung, ohne sie jedoch darauf einzuschränken.

### Beispiel 1

| Reaktanden | Menge (g) |
|---|---|
| NH₄-Acryloyldimethyltaurat | 80 |
| Vinyl-dimethoxyethylendblocked dimethicon (^{®}GP-501, Genesee Pol. Corp.) | 20 |
| t-Butanol | 400 |
| Dilauroylperoxid (Initiator) | 1 |
| Poly-N-Vinylpyrrolidon (^{®}K-15 BASF) | 5 |

Das Polymer wurde nach dem Fällungsverfahren in tert. Butanol hergestellt. Dabei wurden die Monomere in t-Butanol vorgelegt, die Reaktionsmischung inertisiert und anschließend die Reaktion nach Anheizen durch Zugabe von DLP initiiert. Das Polymer wurde durch Absaugen des Lösungsmittels und durch anschließende Vakuumtrocknung isoliert. Das Polymer zeigt in 1 %iger wässriger Lösung eine Viskosität von 45 000 mPas bei leicht opaleszenter Optik. Das Hautgefühl des Gels ist dem silikonfreier Varianten deutlich überlegen.

### Beispiel 2

| Reaktanden | Menge (g) |
|---|---|
| NH₄-Acryloyldimethyltaurat | 70 |
| N-Vinylpyrrolidon | 5 |
| Methacryloxypyldimethicon (^{®}GP-446, Genesee Pol. Corp.) | 15 |
| Isopropanol | 500 |
| Azobisisobutyronitril (Initiator) | 1 |

Das Polymer wurde nach dem Lösungspolymerisationsverfahren in Isopropanol hergestellt. Dabei wurden die Monomere in dem entsprechenden Alkohol gelöst, die Reaktionsmischung inertisiert und anschließend die Reaktion nach Anheizen durch Zugabe von Diazoisobutyronitril initiiert. Die Polymerlösung wurde anschließend eingeengt und mittels Vakuumtrocknung das Polymer isoliert.

### Beispiel 3

| Reaktanden | Menge (g) |
|---|---|
| Acryloyldimethyltaurinsäure (AMPS) | 80 |
| Methacryloxypyl dimethicon (^{®}GP-478, Genesee Pol. Corp.) | 20 |
| Cyclohexan | 200 |
| Wasser | 300 |
| ^{®}Span 80 (Sorbitanester) | 1 |
| Na₂S₂O₈ (Initiator) | 1 |

Das Polymer wurde nach dem Emulsionsverfahren in Wasser hergestellt. Dabei wurden die Monomere in einer Wasser/Cyclohexan unter Verwendung von Span ^{®}80 emulgiert, die Reaktionsmischung mittels N₂ inertisiert und anschließend die Reaktion nach Anheizen durch Zugabe von Natriumperoxodisulfat gestartet. Die Polymeremulsion wurde anschließend eingedampft (Cyclohexan fungiert als Schlepper für Wasser) und dadurch das Polymer isoliert.

### Beispiel 4

| Reaktanden | Menge (g) |
|---|---|
| NH₄-Acryloyldimethyltaurat | 80 |
| Monofunktionalisiertes ethoxyliertes Siloxan (methacrylisch, ^{®}Silvet Y-12867, Witco) | 15 |
| t-Butanol | 300 |
| Dilauroylperoxid | 1 |

Das Polymer wurde nach dem Fällungsverfahren in tert. Butanol hergestellt. Dabei wurden die Monomere in t-Butanol vorgelegt, die Reaktionsmischung inertisiert und anschließend die Reaktion nach Anheizen durch Zugabe von DLP initiiert. Das Polymer wurde durch Absaugen des Lösungsmittels und durch anschließende Vakuumtrocknung isoliert.

Das Polymer zeigte in 1 %iger Lösung in destilliertem Wasser eine klare Optik bei einer Viskosität von 35 000 mPas. Im Vergleich dazu zeigte die silikonfreie Variante bei gleicher Zusammensetzung eine ähnliche Optik und eine Viskosität von 12 000 mPas unter gleichen Messbedingungen. Das Hautgefühl des silikonhaltigen Polymers ist deutlich verbessert gegenüber den Vergleichsstandards.

### Beispiel 5

| Reaktand | Menge (g) |
|---|---|
| Na-Acryloyldimethyltaurat | 50 |
| Monofunktionalisiertes (methacrylisch), ethoxyliertes Siloxan (^{®}Silvet 7608 WITCO) | 45 |
| t-Butanol | 300 |
| Trimethylolpropantriacrylat | 1,8 |
| Aza-bis-amidopropylhydrochlorid | 1 |
| Poly[N-Vinylformamid] | 8 |

Das Polymer wurde nach dem Fällungsverfahren in tert. Butanol hergestellt. Dabei wurden die Monomere in t-Butanol vorgelegt, die Reaktionsmischung inertisiert und anschließend die Reaktion nach Anheizen durch Zugabe von ABAH initiiert. Das Polymer wurde durch Absaugen des Lösungsmittels und durch anschließende Vakuumtrocknung isoliert.

## Patentansprüche

1. Wasserlösliche oder wasserquellbare Copolymere, erhältlich durch radikalische Copolymerisation von
A) Acryloyldimethyltaurinsäure und/oder Acryloyldimethyltauraten,
B) gegebenenfalls einem oder mehreren olefinisch ungesättigten, gegebenenfalls vernetzenden, Comonomeren, die wenigstens ein Sauerstoff-, Stickstoff-, Schwefel- oder Phosphoratom aufweisen und ein Molekulargewicht kleiner 500 g/mol besitzen, und ausgewählt sind aus ungesättigten Carbonsäuren und deren Anhydriden und Salzen, sowie deren Estern mit aliphatischen, olefinischen, cycloaliphatischen, arylaliphatischen oder aromatischen Alkoholen mit einer Kohlenstoffzahl von 1 bis 22, offenkettigen N-Vinylamiden, bevorzugt N-Vinylformamid (VIFA), N-Vinylmethylformamid, N-Vinylmethylacetamid (VIMA) und N-Vinylacetamid; cyclischen N-Vinylamiden (N-Vinyllactame) mit einer Ringgröße von 3 bis 9, bevorzugt N-Vinylpyrrolidon (NVP) und N-Vinylcaprolactam; Amiden der Acryl- und Methacrylsäure, bevorzugt Acrylamid, Methacrylamid, N,N-Dimethyl-acrylamid, N,N-Diethylacrylamid und N,N-Diisopropylacrylamid; alkoxylierten Acryl- und Methacrylamiden, bevorzugt Hydroxyethylmethacrylat, Hydroxymethylmethacrylamid, Hydroxyethylmethacrylamid, Hydroxypropylmethacrylamid und Bernsteinsäuremono-[2-(methacryloyloxy)-ethylester]; N,N-Dimethylaminomethacrylat und Diethylaminomethylmethacrylat; Acryl- und Methacrylamidoglykolsäure; 2- und 4-Vinylpyridin; Vinylacetat; Methacrylsäureglycidylester; Styrol; Acrylnitril; Vinylchlorid; Stearylacrylat; Laurylmethacrylat; Vinylidenchlorid; und/oder Tetrafluorethylen; anorganischen Säuren und deren Salzen und Estern, bevorzugt Vinylphosphonsäure, Vinylsulfonsäure, Allylphosphonsäure und Methallylsulfonsäure,
C) einer oder mehreren mindestens monofunktionellen, zur radikalischen Polymerisation befähigten, siliziumhaltigen Komponente(n), wobei mindestens eine siliziumhaltige Komponente eine Verbindung ausgewählt aus den Formeln 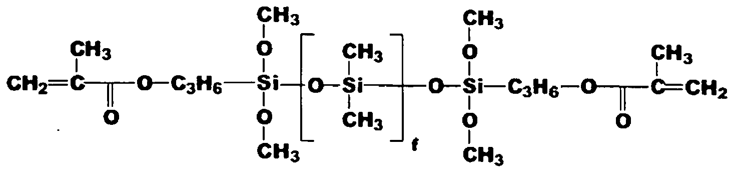 mit f=2 bis 500 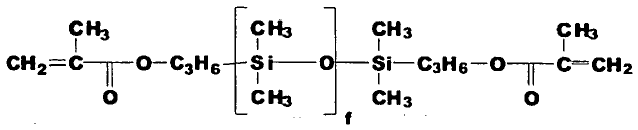 mit f=2 bis 500
und/oder 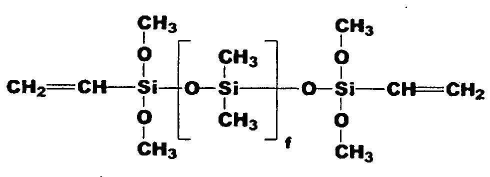 mit f=2 bis 500 ist, wobei die Copolymerisation
D) in Gegenwart mindestens eines polymeren Additivs mit zahlenmittleren Molekulargewichten von 200 g/mol bis 10⁹ g/mol ausgewählt aus Homo- und Copolymeren aus N-Vinylformamid, N-Vinylacetamid, N-Vinylpyrrolidon, Acryloyldimethyltaurinsäure, N-Vinylcaprolactam, N-Vinylmethylacetamid, Acrylamid, Acrylsäure, Methacrylsäure, N-Vinylmorpholid, Hydroxyethylmethacrylat, Diallyldimethylammoniumchlorid (DADMAC) und/oder [2-(Methacryloyloxy)ethyl]trimethylammoniumchlorid (MAPTAC); erfolgt.

2. Copolymere nach Anspruch 1, **dadurch gekennzeichnet, dass** es sich bei der Komponente C) um weitere Verbindungen der Formel (I)
R¹- Z- [(Si(R³R⁴)-O-)_{w}-(Si(R⁵R⁶)-O)ₓ-]- R² (I)
handelt, wobei
R¹ einen Vinyl-, Allyl-, Methallyl-, Methylvinyl-, Acryl-, Methacryl-, Crotonyl-, Senecionyl-, Itaconyl-, Maleinyl-, Fumaryl- oder ein Styrylrest darstellt;
Z eine chemische Brücke, bevorzugt ausgewählt aus -O-, -((C₁ - C₅₀) Alkylen)-,
-((C₆ - C₃₀) Arylen)-, -((C₅ - C₈) Cycloalkylen)-, -((C₁-C₅₀) Alkenylen)-, -(Polypropylenoxid)ₙ-, -(Polyethylenoxid)ₒ-, -(Polypropylenoxid)ₙ(Polyethylenoxid)ₒ-, wobei n und o unabhängig voneinander Zahlen von 0 bis 200 bedeuten und die Verteilung der EO/PO-Einheiten statistisch oder blockförmig sein kann, -((C₁ - C₁₀) Alkyl)-(Si(OCH₃)₂)- und -(Si(OCH₃)₂)-, darstellt;
R³, R⁴, R⁵ und R⁶ unabhängig voneinander -CH₃, -O-CH₃, -C₆H₅ oder -O-C₆H₅ bedeuten;
w, x Zahlen von 0 bis 500 bedeuten, wobei entweder w oder x größer Null sein muss, und
R² einen gesättigten oder ungesättigten, aliphatischen cycloaliphatischen, arylaliphatischen oder aromatischen Rest mit jeweils 1 bis 50 C-Atomen oder eine Gruppe der Formeln -OH, -NH₂, -N(CH₃)₂, -R⁷ oder eine Gruppe-Z-R¹ bedeutet, wobei Z und R¹ die obengenannten Bedeutungen haben und R⁷ eine Gruppe der Formel -O-Si(CH₃)₃, -O-Si(Phenyl)₃, -O-Si(O-Si(CH₃)₃)₂CH₃) und -O-Si(O-Si(Ph)₃)₂Ph) bedeutet.

3. Copolymere nach Anspruch 1 oder 2, **dadurch gekennzeichnet, dass** sie zusätzlich ein oder mehrere Comonomere B) enthalten.

4. Copolymere nach Anspruch 3, **dadurch gekennzeichnet, dass** es sich bei den Comonomeren B um ungesättigte Carbonsäuren, Salze ungesättigter Carbonsäuren, Anhydride ungesättigter Carbonsäuren, Ester ungesättigter Carbonsäuren mit aliphatischen, olefinischen, cycloaliphatischen, arylaliphatischen oder aromatischen Alkoholen mit 1 bis 22 C-Atomen, offenkettige N-Vinylamide, cyclische N-Vinylamide mit einer Ringgröße von 3 bis 9, Amide der Acrylsäure, Amide der Methacrylsäure, Amide substituierter Acrylsäuren, Amide substituierter Methacrylsäuren,
2-Vinylpyridin, 4-Vinylpyridin, Vinylacetat; Styrol, Acrylnitril, Vinylchlorid, Vinylidenchlorid, Tetrafluorethylen, Vinylphosphonsäure oder deren Ester oder Salze, Vinylsulfonsäure oder deren Ester oder Salze, Allylphosphonsäure oder deren Ester oder Salze und/oder Methallylsulfonsäure oder deren Ester oder Salze handelt.

5. Copolymere nach mindestens einem der Ansprüche 1 bis 4, **dadurch gekennzeichnet, dass** die Copolymerisation in Gegenwart mindestens eines polymeren Additivs D) erfolgt,
**dadurch gekennzeichnet, dass** es sich bei den polymeren Additiven D) um Poly(N-Vinylformamide), Poly(N-Vinylcaprolactame) und Copolymere aus N-Vinylpyrrolidon, N-Vinylformamid und/oder Acrylsäure handelt.

6. Copolymere nach mindestens einem der Ansprüche 1 bis 5, **dadurch gekennzeichnet, dass** sie vernetzt sind.

7. Copolymere nach mindestens einem der Ansprüche 1 bis 6, **dadurch gekennzeichnet, dass** sie durch Fällungspolymerisation in tert.-Butanol hergestellt werden.

## Claims

1. A water-soluble or water-swellable copolymer obtainable by free-radical copolymerization of
A) acryloyldimethyltaurine and/or acryloyldimethyltaurates,
B) if desired, one or more olefinically unsaturated, optionally crosslinking comonomers containing at least one oxygen, nitrogen, sulfur or phosphorus atom and possessing a molecular weight of less than 500 g/mol, and selected from unsaturated carboxylic acids and their anhydrides and salts, and also their esters with aliphatic, olefinic, cycloaliphatic, arylaliphatic or aromatic alcohols having a carbon number of from 1 to 22, open-chain N-vinyl amides, preferably N-vinylformamide (VIFA), N-vinylmethylformamide, N-vinylmethylacetamide (VIMA) and N-vinylacetamide; cyclic N-vinyl amides (N-vinyl lactams) with a ring size of 3 to 9, preferably N-vinylpyrrolidone (NVP) and N-vinylcaprolactam; amides of acrylic and methacrylic acid, preferably acrylamide, methacrylamide, N,N-dimethylacrylamide, N,N-diethylacrylamide, and N,N-diisopropylacrylamide; alkoxylated acrylamides and methacrylamides, preferably hydroxyethyl methacrylate, hydroxymethyl-methacrylamide, hydroxyethylmethacrylamide, hydroxypropylmethacrylamide, and mono [2-(methacryloyloxy)ethyl] succinate; N,N-dimethylamino methacrylate; diethylaminomethyl methacrylate; acrylamido- and methacrylamidoglycolic acid; 2- and 4-vinylpyridine; vinyl acetate; glycidyl methacrylate; styrene; acrylonitrile; vinyl chloride; stearyl acrylate; lauryl methacrylate; vinylidene chloride; and/or tetrafluoroethylene; inorganic acids and their salts and esters, preferably vinylphosphonic acid, vinylsulfonic acid, allylphosphonic acid, and methallylsulfonic acid,
C) one or more at least monofunctional, silicon-containing components capable of free-radical polymerization, at least one silicon-containing component being a compound selected from the formulae 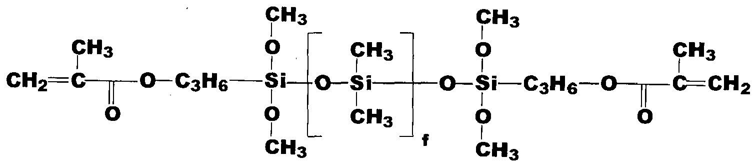 where f = 2 to 500 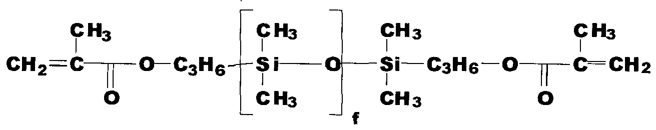 where f = 2 to 500
and/or 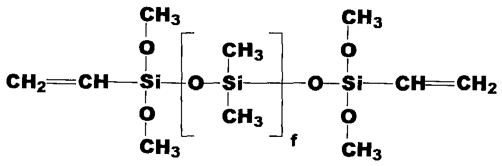 where f = 2 to 500, the copolymerization
D) taking place in the presence of at least one polymeric additive having number-average molecular weights of from 200 g/mol to 10⁹ g/mol selected from homopolymers and copolymers of N-vinylformamide, N-vinylacetamide, N-vinylpyrrolidone, acryloyldimethyltaurine, N-vinylcaprolactam, N-vinylmethylacetamide, acrylamide, acrylic acid, methacrylic acid, N-vinylmorpholide, hydroxy ethyl methacrylate, diallyldimethylammonium choride (DADMAC) and/or [2-(methacryloyloxy)ethyl]trimethylammonium chloride (MAPTAC).

2. The copolymer as claimed in claim 1, wherein component C) comprises further compounds of the formula (I)
R¹-Z-[(Si(R³R⁴)-O-)_{w}-(Si(R⁵R⁶)-O)ₓ-]-R² (I)
where
R¹ represents a vinyl, allyl, methallyl, methylvinyl, acryloyl, methacryloyl, crotonyl, senecionyl, itaconyl, maleyl, fumaryl or styryl radical;
Z is a chemical bridge, preferably selected from -O-, -((C₁-C₅₀)alkylene)-, - ((C₆-C₃₀)arylene)-, - ((C₅-C₈)cycloalkylene)-,-((C₁-C₅₀)alkenylene) -, -(polypropylene oxide)ₙ-, -(polyethylene oxide)ₒ-, -(polypropylene oxide)ₙ(polyethylene oxide)ₒ-, where n and o independently of one another denote numbers from 0 to 200 and the distribution of the EO/PO units can be random or in the form of blocks, -((C₁-C₁₀)alkyl)-(Si(OCH₃)₂)- and -(Si(OCH₃)₂)-;
R³, R⁴, R⁵, and R⁶ independently of one another denote-CH₃, -O-CH₃, -C₆H₅ or
-O-C₆H₅;
w, x denote numbers from 0 to 500, it being necessary for either w or x to be greater than zero, and
R² is a saturated or unsaturated, aliphatic, cycloaliphatic, arylaliphatic or aromatic radical having in each case 1 to 50 carbon atoms or is a group of the formulae -OH, -NH₂, -N(CH₃)₂, -R⁷ or a group -Z-R¹, where Z and R¹ have the definitions stated above and R⁷ is a group of the formula -O-Si(CH₃)₃, -O-Si(phenyl)₃, -O-Si(O-Si(CH₃)₃)₂CH₃) and -O-Si(O-Si(Ph)₃)₂Ph).

3. The copolymer as claimed in claim 1 or 2, further containing one or more comonomers B).

4. The copolymer as claimed in claim 3, wherein the comonomers B comprise unsaturated carboxylic acids, salts of unsaturated carboxylic acids, anhydrides of unsaturated carboxylic acids, esters of unsaturated carboxylic acids with aliphatic, olefinic, cycloaliphatic, arylaliphatic or aromatic alcohols having 1 to 22 carbon atoms, open-chain N-vinyl amides, cyclic N-vinyl amides having a ring size of from 3 to 9, amides of acrylic acid, amides of methacrylic acid, amides of substituted acrylic acids, amides of substituted methacrylic acids, 2-vinylpyridine, 4-vinylpyridine, vinyl acetate; styrene, acrylonitrile, vinyl chloride, vinylidene chloride, tetrafluoroethylene, vinylphosphonic acid or the esters or salts thereof, vinylsulfonic acid or the esters or salts thereof, allylphosphonic acid or the esters or salts thereof and/or methallylsulfonic acid or the esters or salts thereof.

5. The copolymer as claimed in at least one of claims 1 to 4, wherein the copolymerization takes place in the presence of at least one polymeric additive D), wherein the polymeric additives D) are poly(N-vinylformamides), poly(N-vinylcaprolactams), and copolymers of N-vinylpyrrolidone, N-vinylformamide and/or acrylic acid.

6. The copolymer as claimed in at least one of claims 1 to 5, which is crosslinked.

7. The copolymer as claimed in at least one of claims 1 to 6, prepared by precipitation polymerization in tert-butanol.

## Revendications

1. Copolymères solubles dans l'eau ou gonflants dans l'eau, pouvant être obtenus par copolymérisation radicalaire de
A) de l'acide acryloyldiméthyltaurique et/ou des taurates d'acryloyldiméthyle,
B) éventuellement un ou plusieurs comonomères oléfiniquement insaturés, éventuellement réticulants, qui comprennent au moins un atome d'oxygène, d'azote, de soufre ou de phosphore et qui présentent un poids moléculaire inférieur à 500 g/mol, et qui sont choisis parmi les acides carboxyliques insaturés et leurs anhydrides et sels, ainsi que leurs esters avec des alcools aliphatiques, oléfiniques, cycloaliphatiques, arylaliphatiques ou aromatiques ayant un nombre de carbone de 1 à 22, les N-vinylamides à chaîne ouverte, de préférence le N-vinylformamide (VIFA), le N-vinylméthylformamide, le N-vinylméthylacétamide (VIMA) et le N-vinylacétamide ; les N-vinylamides cycliques (N-vinyllactames) d'une taille de cycle de 3 à 9, de préférence la N-vinylpyrrolidone (NVP) et le N-vinylcaprolactame ; les amides de l'acide acrylique et méthacrylique, de préférence l'acrylamide, le méthacrylamide, le N,N-diméthylacrylamide, le N,N-diéthylacrylamide et le N,N-diisopropylacrylamide ; les acryl- et méthacrylamides alcoxylés, de préférence le méthacrylate d'hydroxyéthyle, l'hydroxyméthylméthacrylamide, l'hydroxyéthylméthacrylamide, l'hydroxypropylméthacrylamide et l'ester mono-[2-(méthacryloyloxy)-éthylique] de l'acide succinique ; l'aminométhacrylate de N,N-diméthyle et le méthacrylate de diéthylaminométhyle ; l'acide acryl- et méthacrylamidoglycolique ; la 2- et 4-vinylpyridine ; l'acétate de vinyle ; l'ester glycidylique de l'acide méthacrylique ; le styrène ; l'acrylonitrile ; le chlorure de vinyle ; l'acrylate de stéaryle ; le méthacrylate de lauryle ; le chlorure de vinylidène ; et/ou le tétrafluoroéthylène ; les acides inorganiques et leurs sels et esters, de préférence l'acide vinylphosphonique, l'acide vinylsulfonique, l'acide allylphosphonique et l'acide méthallylsulfonique,
C) un ou plusieurs composants contenant du silicium, au moins monofonctionnels, aptes à la polymérisation radicalaire, au moins un composant contenant du silicium étant un composé choisi parmi les formules 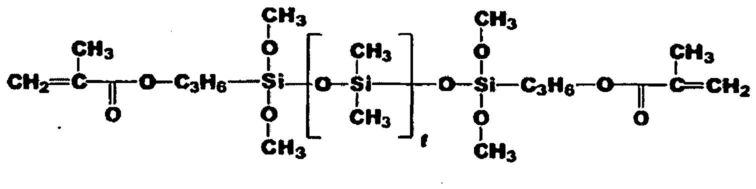 avec f = 2 à 500, 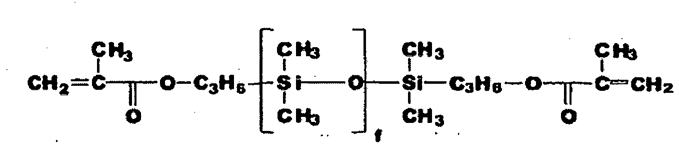 avec f = 2 à 500
et/ou 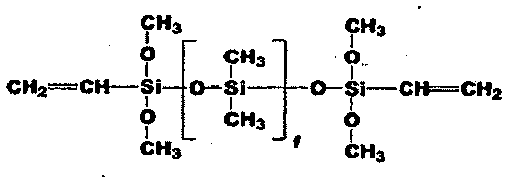 avec f = 2 à 500,
D) la copolymérisation ayant lieu en présence d'au moins un additif polymère de poids moléculaire moyen en nombre de 200 g/mol à 10⁹ g/mol, choisi parmi les homo- et copolymères de N-vinylformamide, N-vinylacétamide, N-vinylpyrrolidone, acide acryloyldiméthyltaurique, N-vinylcaprolactame, N-vinylméthylacétamide, acrylamide, acide acrylique, acide méthacrylique, N-vinylmorpholide, méthacrylate d'hydroxyéthyle, chlorure de diallyldiméthylammonium (DADMAC) et/ou chlorure de [2-(méthacryloyloxy)éthyl]triméthylammonium (MAPTAC).

2. Copolymères selon la revendication 1, **caractérisés en ce que** le composant C) consiste en des composés supplémentaires de formule (I)
R¹-Z-[(Si(R³R⁴)-O-)_{w}-(Si(R⁵R⁶)-O)ₓ-]-R² (I)
dans laquelle
R¹ représente un radical vinyle, allyle, méthallyle, méthylvinyle, acryle, méthacryle, crotonyle, sénécionyle, itaconyle, maléinyle, fumaryle ou styryle ;
Z représente un pont chimique, de préférence choisi parmi -O-, -(alkylène en (C₁-C₅₀))-, -(arylène en (C₆-C₃₀))-, -(cycloalkylène en (C₅-C₈))-, -(alcénylène en (C₁-C₅₀))-, -(oxyde de polypropylène)ₙ-, -(oxyde de polyéthylène)ₒ-, -(oxyde de polypropylène)ₙ(oxyde de polyéthylène)ₒ-, n et o signifiant indépendamment l'un de l'autre des nombres de 0 à 200 et la distribution des unités EO/PO pouvant être statistique ou séquencée, -(alkyle en (C₁-C₁₀))-(Si(OCH₃)₂)- et -(Si(OCH₃)₂)- ;
R³, R⁴, R⁵ et R⁶ signifient indépendamment les uns des autres -CH₃, -O-CH₃, -C₆H₅ ou -O-C₆H₅ ;
w, x signifient des nombres de 0 à 500, w ou x devant être supérieur à zéro, et
R² signifie un radical saturé ou insaturé, aliphatique, cycloaliphatique, arylaliphatique ou aromatique, contenant à chaque fois 1 à 50 atomes C, ou un groupe de formule -OH, -NH₂, -N(CH₃)₂, -R⁷ ou un groupe -Z-R¹, Z et R¹ ayant les significations indiquées précédemment, et
R⁷ signifiant un groupe de formule -O-Si(CH₃)₃, -O-Si(phényle)₃, -O-Si(O-Si(CH₃)₃)₂CH₃ et -O-Si(O-Si(Ph)₃)₂Ph.

3. Copolymères selon la revendication 1 ou 2, **caractérisés en ce qu'**ils contiennent en outre un ou plusieurs comonomères B).

4. Copolymères selon la revendication 3, **caractérisés en ce que** les comonomères B sont des acides carboxyliques insaturés, des sels d'acides carboxyliques insaturés, des anhydrides d'acides carboxyliques insaturés, des esters d'acides carboxyliques insaturés avec des alcools aliphatiques, oléfiniques, cycloaliphatiques, arylaliphatiques ou aromatiques de 1 à 22 atomes C, des N-vinylamides à chaîne ouverte, des N-vinylamides cycliques d'une taille de cycle de 3 à 9, des amides de l'acide acrylique, des amides de l'acide méthacrylique, des amides d'acides acryliques substitués, des amides d'acides méthacryliques substitués, la 2-vinylpyridine, la 4-vinylpyridine, l'acétate de vinyle ; le styrène, l'acrylonitrile, le chlorure de vinyle, le chlorure de vinylidène, le tétrafluoroéthylène, l'acide vinylphosphonique ou ses esters ou sels, l'acide vinylsulfonique ou ses esters ou sels, l'acide allylphosphonique ou ses esters ou sels et/ou l'acide méthallylsulfonique ou ses esters ou sels.

5. Copolymères selon au moins l'une quelconque des revendications 1 à 4, **caractérisés en ce que** la copolymérisation a lieu en présence d'au moins un additif polymère D), l'additif polymère D) consistant en des poly(N-vinylformamides), des poly(N-vinylcaprolactames) et des copolymères de N-vinylpyrrolidone, N-vinylformamides et/ou acide acrylique.

6. Copolymères selon au moins l'une quelconque des revendications 1 à 5, **caractérisés en ce qu'**ils sont réticulés.

7. Copolymères selon au moins l'une quelconque des revendications 1 à 6, **caractérisés en ce qu'**ils sont fabriqués par polymérisation par précipitation dans du tert.-butanol.
